# FASCICULE DE BREVET EUROPEEN

(11) **EP 1 301 167 B1**
(45) Date de publication et mention de la délivrance du brevet: **20.09.2006**
(21) Numéro de dépôt: 01936736.6
(22) Date de dépôt: 13.06.2001
(51) Int. Cl.: A61K 8/33, A61Q 13/00, C11D 3/20, C11D 3/50, C11B 9/00, C07C 43/303

(54) **UTILISATION DU (1-ETHOXYETHOXY)CYCLODODECANE DANS UNE COMPOSITION PARFUMANTE A TITRE D'AGENT FIXATEUR ET/OU AMPLIFICATEUR DE PARFUM**
VERWENDUNG VON (1-ETHOXYETHOXY)CYCLODODECAN IN EINER PARFÜMZUSAMMENSETZUNG ALS FIXIERMITTEL UND/ODER PARFÜMVERSTÄRKER
USE OF (1-ETHOXYETHOXY)CYCLODODECANE IN A PERFUME COMPOSITION AS PERFUME FIXATIVE AND/OR ENHANCER

(30) Priorité: 10.07.2000 CH 20001354
(43) Date de publication de la demande: 16.04.2003
(73) Titulaire: FIRMENICH SA, 1211 Genève 8 (CH)
(72) Inventeur: MARGOT, Christian, CH-1182 Gilly (CH); BLANC, Pierre-Alain, CH-1263 Crassier (CH)
(74) Mandataire: Salvaterra-Garcia, Maria de Lurdes
(86) Numéro de dépôt international: PCT/IB2001/001048
(87) Numéro de publication internationale: WO 2002/003941

(56) Documents cités:
- FR-A- 1 393 647
- US-A- 3 993 697
- US-A- 4 990 495

## Description

### Domaine technique

La présente invention a trait au domaine de la parfumerie. Elle concerne plus particulièrement l'utilisation du (1-éthoxyéthoxy)cyclododécane dans une composition parfumante, à titre d'agent fixateur et/ou amplificateur de parfum.

Le (1-éthoxyéthoxy)cyclododécane, bien que possédant une odeur faiblement perceptible ou, pour beaucoup de personnes, même non détectable, se révèle présenter en composition un intérêt tout à fait remarquable. Nous avons établi, en effet, que ledit composé fixe et intensifie le caractère odorant des compositions auxquelles il est ajouté, tout en leur apportant une jolie note odorante boisée qui n'est pas nécessairement perçue lorsque le composé est évalué individuellement.

### Technique antérieure

Le brevet US 3,993,697 décrit à travers une formule générale un certain nombre d'acétals substitués par un groupe cycloalkyle présentant une odeur de type ambré et boisé. Bien que le (1-éthoxyéthoxy)cyclododécane soit compris dans la formule générale divulguée par ce brevet, et qu'il soit mentionné dans la liste des composés odorants, il est également cité comme ne faisant pas partie des structures les plus odorantes et les plus appropriées pour une utilisation à titre d'ingrédients parfumants. L'odeur du (1-éthoxyéthoxy)cyclododécane n'est d'ailleurs pas spécifiquement décrite dans ce document, et bien qu'une méthode de synthèse soit divulguée pour l'ensemble des composés tombant sous la formule générale, la préparation du composé en question n'est spécifiée dans aucun exemple, et il n'est pas non plus utilisé dans les exemples d'application en parfumerie décrits. Il apparaît donc au vu de ce document que le (1-éthoxyéthoxy)cyclododécane n'a pas été perçu comme très intéressant du point de vue olfactif et on peut même se poser la question de savoir si les inventeurs l'ont en main. Des tests décrits dans les exemples présentés plus bas effectués par des experts parfumeurs ou des juges, confirmeront le fait que le composé se trouve être très faiblement odorant et même totalement dénué d'odeur pour une certaine partie de la population.

### Exposé de l'invention

Or, malgré le fait que l'odeur du (1-éthoxyéthoxy)cyclododécane ne soit perceptible que par une minorité de personnes, nous avons maintenant découvert que, de façon tout à fait surprenante, ledit composé procure un effet technique à la fois nouveau et inattendu, reconnu de façon générale lorsque celui-ci est utilisé en composition, même par les personnes touchées d'anosmie vis-à-vis du composé pur. Nous avons ainsi pu établir que le (1-éthoxyéthoxy)cyclododécane peut de façon très avantageuse être utilisé dans une composition parfumante à titre d'agent fixateur et/ou amplificateur de parfum tout en ajoutant à la fragrance de la composition une jolie note boisée. On entend ici par agent fixateur un ingrédient capable d'améliorer un parfum par un effet technique complexe qui se traduit par un effet à la fois d'arrondi, de ténacité, de dimension ou volume ou encore de richesse olfactive apportée à l'odeur d'une composition.

Outre son action fixatrice, le (1-éthoxyéthoxy)cyclododécane a également pour effet d'amplifier ou de renforcer certaines notes odorantes de la composition à laquelle il est ajouté, telles que en particulier, les notes de type musqué et aromatique.

Il a été également observé, que, alors que la plupart des personnes ne sent pas le composé en tant que tel, celui-ci apporte de façon avantageuse aux compositions parfumantes auxquelles il est ajouté, une très jolie note odorante de type boisé perçue de façon générale. On peut d'ailleurs souligner le fait que l'effet produit par le composé de l'invention est tout à fait différent de celui des composés préférés décrits dans le brevet US 3,993,697. Par exemple le (méthoxyméthoxy)cyclododécane, un composé particulièrement apprécié selon le document cité pour ses qualités olfactives, possède une odeur très puissante de type boisé et ambré. Ce composé doit être utilisé en très faible quantité en raison de sa puissance et ne donne aucun volume ou dimension aux compositions auxquelles il est ajouté. L'effet du (1-éthoxyéthoxy)cyclododécane qui, pour sa part, se révèle pratiquement inutile, est beaucoup plus subtil en composition et outre une odeur de type boisé, qui représente un intérêt particulier dans le domaine de la parfumerie, il apporte la fixation telle que définie plus haut et l'amplification du parfum, conférant ainsi une nouvelle dimension à l'odeur de la composition qui le contient. En d'autres termes, les compositions auxquelles on ajoute le (1-éthoxyéthoxy)cyclododécane acquièrent une qualité de parfum qui se traduit à la fois par un arrondi de la note odorante, une dimension et une intensité difficiles à atteindre avec des ingrédients parfumants classiquement connus pour leur odeur boisée.

Ainsi, grâce à sa qualité d'agent fixateur et/ou amplificateur, le (1-éthoxyéthoxy)cyclododécane, contrairement à un ingrédient parfumant "classique" n'a pas pour simple effet d'ajouter une note odorante particulière à un mélange de façon à nuancer l'odeur de celui-ci. L'effet technique produit par le composé de l'invention est de nature plus complexe, comme cela a été décrit plus haut. Les tests comparatifs présentés plus loin témoignent d'ailleurs de l'effet du composé de l'invention en composition, par rapport à des ingrédients parfumants "classiques" connus pour leur odeur boisée. Dans le cas présent, il s'agit donc véritablement d'un effet technique nouveau et très intéressant pour les parfumeurs.

Les compositions parfumantes auxquelles on ajoute le composé de l'invention de façon à donner à la fois intensité, arrondi, richesse et ténacité, sont des mélanges d'ingrédients parfumants, de solvants ou adjuvants d'usage courant en parfumerie. La nature et la variété de ces co-ingrédients n'appellent pas une description plus détaillée ici, qui ne saurait d'ailleurs être exhaustive, l'homme du métier étant à même de les choisir de par ses connaissances générales et en fonction de la nature du produit à parfumer et de l'effet olfactif recherché.

Ces ingrédients parfumants appartiennent à des classes chimiques aussi variées que les alcools, aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres ouvrages de nature similaire.

Les compositions contenant le (1-éthoxyéthoxy)cyclododécane selon l'invention se prêtent aussi bien à une utilisation en parfumerie fine, dans les parfums, eaux de toilette ou lotions après-rasage, qu'à d'autres emplois courants en parfumerie tels que le parfumage des savons, gels de douche ou de bain, de produits d'hygiène ou de produits de traitement des cheveux comme les shampooings ou après shampooings, ou d'autres produits de soins capillaires, ainsi que des déodorants corporels et déodorants d'ambiance, ou encore des préparations cosmétiques.

Les compositions parfumantes de l'invention peuvent également être employées dans des applications telles que détergents liquides ou solides destinées au traitement des textiles, adoucissants textiles ou encore dans des compositions détergentes ou produits d'entretien destinés au nettoyage de la vaisselle ou de surfaces variées, à usages domestiques ou industriels.

Les proportions dans lesquelles le (1-éthoxyéthoxy)cyclododécane peut être incorporé dans les produits divers susmentionnés varient dans une gamme de valeurs étendue. Ces valeurs dépendent de la nature de l'article ou produit que l'on veut parfumer et de l'effet recherché, à savoir intensité de certaines notes odorantes, arrondi, volume, ténacité, richesse de l'odeur finale, ainsi que de la nature des coingrédients dans une composition donnée. Il a en effet été mentionné plus haut en particulier que le (1-éthoxyéthoxy)cyclododécane en composition était particulièrement avantageux pour renforcer les notes musquées et aromatiques.

A titre d'exemple, on peut citer des concentrations typiques de l'ordre de 2 à 30% en poids du composé par rapport au poids de la composition parfumante dans laquelle il est incorporé.

Le (1-éthoxyéthoxy)cyclododécane peut être préparé en une étape à partir du cyclododécanol en présence d'éthyl-vinyl-éther et d'un catalyseur acide. Les conditions spécifiques de la réaction seront décrites en détail dans l'un des exemples qui suivent.

L'invention sera maintenant décrite de façon plus détaillée à l'aide des exemples suivants dans lesquels les températures sont indiquées en degrés Celsius et les abréviations ont le sens usuel dans l'art.

### Manières de réaliser l'invention

### Exemple 1

### Préparation du (1-éthoxyéthoxy)cyclododécane

Un mélange de 300 g (1,62 mol) de cyclododécanol (origine : Fluka) et 1,25 1 de méthyl-*tert*-butyle éther a été versé dans un réacteur de 3 1 et dissout par chauffage à 35°. Après avoir refroidi à 10°, une recristallisation partielle de l'alcool a été observée (cristaux fins). Après avoir ajouté environ 20 mg d'acide *p*-toluènesulfonique, 240 ml d'éthyl-vinyl-éther ont été introduits au moyen d'une ampoule à addition en 2 h, tout en maintenant la température entre 10 et 15°. La température a été maintenue à 15° dès la fin de l'addition. Après un lavage à l'aide de 100 ml de solution aqueuse de NaOH à 5%, on a laissé décanter. Le solvant a été évaporé sous vide en maintenant la température inférieure ou égale à 15°. Une distillation sous vide poussé (Eb 135°, 1,3 Pa) a permis d'obtenir 281 g de produit incolore dont la pureté était de 98,5% avec un rendement de 70%.

Données analytiques :
IR : 2933(s) ; 1471(*m*) ; 1445(*w*) ; 1376(*w*) ; 1130, 1100(*s*)
SM 256(M+, <1%) : 241(1%) ; 227(5%) ; 73(100%)
¹H-RMN(360MHz, CDCl₃) : 4,73(*q*, *J*=5,5, 1H) ; 3,72(*hept*, *J*=4,0, 1H) ; 3,6(*m*, 2H) ; 1,6(*m*, 2H) ; 1,3-1,5(*m*, 20H) ; 1,30(*d*, *J*=5,5, 3H) ; 1,20(*t*, *J*=7,1, 3H).
¹³C-NMR(90MHz, CDCl₃) : 98,3(*d*) ; 73,5(*d*) ; 59,9(*t*) ; 30,2(*t*) ; 29,5(*t*) ; 24,6(2*t*) ; 24,1(*t*) ; 23,5(*t*) ; 23,4(*t*) ; 23,2(*t*) ; 23,1(*t*) ; 21,2(*t*) ; 20,9(*q*) ; 20,7(*t*) ; 15,4(*q*).

### Exemple 2

### Comparaison de l'effet du (1-éthoxyéthoxy)cyclododécane seul avec l'effet de l'Iso E super [1-(octahydro-2,3,8,8-tétraméthyl-2-naphtalényl-1-éthanone ; origine : International Flavors and Fragrances, USA] seul

Deux ensembles de linge (70% coton et 30% synthétique) contenant chacun 18 lavettes de 30x30 cm et du linge divers ont été lavés séparément à 40° dans une machine à laver (Miele, modèle semi professionnel S5425), sans prélavage, à l'aide de 85 g d'une base détergente de type VIA Taed Sensitive (origine : Lever, Stockholm) comprenant respectivement 0,2% de (1-éthoxyéthoxy)cyclododécane dans un cas, et 0,2% d'Iso E super dans l'autre.

Un ensemble de 40 juges a évalué l'intensité de la note boisée du (1-éthoxyéthoxy)cyclododécane en comparaison de l'Iso E super sur une échelle continue variant de 1 (plus faible intensité) à 7 (plus forte intensité), d'une part de la poudre de lavage, puis du linge sec (24 h de séchage à environ 60% d'humidité relative et une température de 21 °) et enfin du linge humide.

Le tableau suivant indique le nombre de juges ayant choisi certaines plages d'intensité de la note boisée pour chacun des 2 ingrédients testés :

**Tableau 1**

| | *Poudre* | | *Linge mouillé* | | *Linge sec* | |
|---|---|---|---|---|---|---|
| **Intensité** | **Nombre de juges** | | | | | |
| | (1-éthoxyéthoxy) cyclododécane | Iso E super | (1-éthoxyéthoxy) cyclododécane | Iso E super | (1-éthoxyéthoxy) cyclododécane | Iso E super |
| **<2** | 11 | 2 | 7 | 4 | 19 | 7 |
| **2-3** | 9 | 2 | 9 | 3 | 8 | 3 |
| **3-4** | 1 | 5 | 9 | 0 | 0 | 2 |
| **4-5** | 10 | 10 | 4 | 7 | 6 | 7 |
| **5-6** | 3 | 14 | 8 | 16 | 6 | 12 |
| **≥6** | 6 | 7 | 3 | 10 | 1 | 9 |

Il apparaît de façon claire au vu de ce tableau que deux populations se distinguent nettement en ce qui concerne la perception du (1-éthoxyéthoxy)cyclododécane. En effet, contrairement à l'Iso E super qui de façon générale est bien perçu par la plupart des panélistes, on remarque qu'un certain nombre de ces personnes ne perçoivent pas ou peu le composé de l'invention (notes entre 1 et 3), et qu'il existe à l'inverse certaines personnes qui le perçoivent mieux (notes entre 5 et 6).

### Exemple 3

### Exemple comparatif entre le (1-éthoxyéthoxy)cyclododécane en composition et deux composés à connotation boisée

L'exemple suivant compare l'effet en composition du (1-éthoxyéthoxy)cyclododécane avec des composés connus pour conférer une note boisée aux compositions auxquelles ils sont ajoutés, à savoir l'iso E super [1-(octahydro-2,3,8,8-tétraméthyl-2-naphtalényl)-1-éthanone ; origine : International Flavors and Fragrances, USA], et le Vertofix (origine : International Flavors and Fragrances, USA).
Les deux composés cités en comparaison du composé de l'invention sont couramment utilisés dans le domaine de la parfumerie.

Un panel d'experts parfumeurs a évalué dans un test à l'aveugle, d'une part la préférence et d'autre part la note boisée des 3 composés respectivement dans deux compositions.

La composition A était constituée des ingrédients suivants :

| Ingrédients | Parties en poids |
|---|---|
| Acétate de bornyle à 10% * | 15 |
| Acétate de géranyle à 10% * | 35 |
| Acétate de linalyle | 70 |
| Méthyle nonyle acétaldéhyde à 10% * | 20 |
| Glycolate d'amyle allyle à 10% * | 15 |
| Anthranilate de méthyle | 5 |
| Essence d'armoise | 10 |
| Bacdanol ® ¹⁾ | 25 |
| Essence de bergamote | 15 |
| Essence de cannelle de Ceylan à 10% * | 70 |
| Essence de castoreum à 10% * | 30 |
| Citral ²⁾ à 10% * | 20 |
| Coumarine | 240 |
| Essence de cumin | 30 |
| Dihydromyrcénol ³⁾ | 270 |
| Eugénol à 10% * | 15 |
| Essence de fleur d'oranger | 30 |
| Galaxolide ® ⁴⁾ | 1100 |
| Géraniol | 10 |
| Essence de lavande | 30 |
| Essence de lavandin grosso | 60 |
| Lilial ® ⁵⁾ | 230 |
| Linalol | 70 |
| Lyral ® ⁶⁾ | 170 |
| Essence de menthe crêpue à 10% * | 30 |
| Iso-méthyl-alpha-ionone | 100 |
| Essence de thym blanc à 10% * | 25 |
| Tonalide ® ⁷⁾ | 750 |
| Triplal ® ⁸⁾ à 1% * | 20 |
| Vanilline | 90 |
| Total | 3600 |

| | |
|---|---|
| * dans le dipropylène glycol 1) 2-éthyl-4-(2,2,3-triméthyl-3-cyclopentèn-1-yl)-2-buténol ; origine: International Flavors and Fragrances 2) origine : Firmenich SA, Genève, Suisse 3) origine : International Flavors and Fragrances, USA 4) 1,3,4,6,7,8-hexahydro-4,6,6,7,8,8-hexaméthyl-cyclopenta-y-2-benzopyrane ; origine : International Flavors and Fragrances, USA 5) 3-(4-tert-butylphényl)-2-méthylpropananl ; origine : Givaudan-Roure SA, Genève, Suisse 6) mélange de 4-(4-hydroxy-4-méthylpentyl)-3-cyclohexène-1-carbaldéhyde et 3-(4-hydroxy-4-méthylpentyl)-3-cyclohexène-1-carbaldéhyde ; origine : International Flavors and Fragrances, USA 7) (5,6,7,8-tétrahydro-3,5,5,6,8,8-hexaméthyl-2-naphtyl)-1-éthanone ; Polak's Frutal Works, Hollande 8) 2,4-diméthyl-3-cyclohexèn-1-carboxaldéhyde ; origine : International Flavors and Fragrances | |

A cette composition de base on a ajouté respectivement 700 parties en poids de chacun des 3 ingrédients à tester et les trois compositions ainsi obtenues ont été évaluées à l'aveugle par un panel de 9 experts parfumeurs, dont 2 seulement avaient perçu la note boisée du (1-éthoxyéthoxy)cyclododécane seul.

La composition B était constituée des ingrédients suivants :

| Ingrédients | Parties en poids |
|---|---|
| Acétate de carbinol | 30 |
| Acétate de dodécyle | 50 |
| Acétate de géranyle | 10 |
| Acétate de styrallyle | 10 |
| Aldéhyde hexylcinnamique | 150 |
| γ-Undécalactone | 10 |
| Benzylacétone | 30 |
| Acétate de verdyle | 70 |
| Coumarine | 5 |
| α-Damascone à 10% * | 30 |
| Dihydromyrcénol ¹⁾ | 70 |
| Habanolide ® ²⁾ | 140 |
| Iralia ® ³⁾ | 40 |
| Essence de Lavandin grosso | 25 |
| 1-(5,5-Diméthyl-1-cyclohexen-1-yl)-4-penten-1-one ⁴⁾ à 10% * | 10 |
| Phénéthylol | 100 |
| Salicylate d'hexyle | 250 |
| Undécavertol ® ⁵⁾ | 10 |
| Essence de violette | 20 |
| Wardia ® ⁶⁾ | 40 |
| Total | 1100 |

| | |
|---|---|
| * dans le dipropylèneglycol 1) origine : International Flavors and Fragrances 2) pentadécenolide ; origine : Firmenich SA, Genève, Suisse 3) mélange d'isomères de méthylionones ; origine : Firmenich SA, Genève, Suisse 4) origine : Firmenich SA, Genève, Suisse 5) 4-méthyl-3-decen-5-ol ; origine : Givaudan-Roure, Vernier, Suisse 6) origine : Firmenich SA; Genève, Suisse | |

A cette composition de base on a ajouté respectivement 300 parties en poids de chacun des 3 ingrédients à tester et les trois compositions ainsi obtenues ont été évaluées à l'aveugle par un panel de 7 experts parfumeurs dont seulement 2 avaient décrits le (1-éthoxyéthoxy)cyclododécane comme ayant une odeur boisée.

Les 2 tableaux suivants indiquent, pour chacun des 3 composés, le nombre d'experts parfumeurs ayant classé en première, deuxième et respectivement troisième position chacun des 3 échantillons qui leur ont été présentés sur des critère de préférence et de note boisée.

**Tableau 2**

| ***COMPOSITION A*** | | | | | | |
|---|---|---|---|---|---|---|
| **Ingrédient ajouté** | *Préférence* | | | *Note boisée* | | |
| | 1ère position | 2ème position | 3ème position | 1ère position | 2ème position | 3ème position |
| (1-éthoxyéthoxy)cyclododécane | **6** | 2 | 1 | **4** | 3 | 2 |
| Iso E super | 3 | **1** | 5 | **4** | 3 | 2 |
| Vertofix | 0 | 6 | 3 | **1** | 3 | 5 |

**Tableau 3**

| ***COMPOSITION B*** | | | | | | |
|---|---|---|---|---|---|---|
| **Ingrédient ajouté** | *Préférence* | | | *Note boisée* | | |
| | 1ère position | 2ème position | 3ème position | 1ère position | 2ème position | 3ème position |
| (1-éthoxyéthoxy)cyclododécane | **4** | 0 | 3 | **3** | 2 | 2 |
| Iso E super | 1 | 3 | 3 | **3** | 2 | 2 |
| vertofix | **2** | 4 | 1 | 1 | 3 | 3 |

Il ressort de ces 2 tableaux, d'une part que le (1-éthoxyéthoxy)cyclododécane est préféré par une majorité des parfumeurs (6/9 et 4/7) aux deux autres composés, connus dans l'art antérieur et utilisés pour conférer des effets olfactifs du même type. Par ailleurs, concernant la note boisée en particulier, il apparaît que le composé de l'invention, alors que peu ou pas détecté par plus de 50% des personnes, se trouve tout à fait comparable pour la note boisée qui se révèle en composition, aux composés généralement utilisés et qui eux sont perçus même purs par la totalité des personnes.

### Exemple 4

### Préparation d'une composition parfumante pour une eau de toilette pour homme à caractère boisé et herbacé

On a préparé une composition de base destinée à une eau de toilette masculine à partir des ingrédients suivants :

| Ingrédients | Parties en poids |
|---|---|
| Acétate de linalyle | 360 |
| Ambrinol à 10% * | 20 |
| Essence de Citron Sfuma | 600 |
| Coumarine | 60 |
| α-Damascone à 10% * | 60 |
| Dihydromyrcénol ¹⁾ | 660 |
| Essence d'estragon | 20 |
| Farenal ²⁾ à 10% * | 30 |
| Polysantol ® ³⁾ | 40 |
| Géraniol | 40 |
| Essence de géranium de Chine | 120 |
| Hedione ® HC ⁴⁾ | 300 |
| Essence de laurier | 10 |
| Linalol | 200 |
| Lyral ® ⁵⁾ | 100 |
| Muscenone ⁶⁾ | 100 |
| Trans-1-(2,2,6-triméthyl-1-cyclohexyl)-3-hexanol ⁷⁾ | 50 |
| Tonalide ® ⁸⁾ | 410 |
| Vanilline | 20 |
| Total | 3200 |

| | |
|---|---|
| * dans le dipropylèneglycol 1) origine : International Flavors and Fragrances, USA 2) 2,6,10-triméthyl-9-undécénal ; origine : Haarmann & Reimer 3) 3,3-diméthyl-5-(2,2,3-triméthyl-3-cyclopenten-1-yl)-4-penten-2-ol ; origine : Firmenich SA, Genève, Suisse 4) dihydrojasmonate de méthyle à haute teneur en isomère cis ; origine : Firmenich SA, Genève, Suisse 5) mélange de 4-(4-hydroxy-4-méthylpentyl)-3-cyclohexène-1-carbaldéhyde et 3-(4-hydroxy-4-méthylpentyl)-3-cyclohexène-1-carbaldéhyde ; origine : International Flavors and Fragrances, USA 6) 3-méthyl-cyclopentadécenone ; origine : Firmenich SA, Genève, Suisse 7) origine : Firmenich SA, Genève, Suisse 8) (5,6,7,8-tétrahydro-3,5,5,6,8,8-hexaméthyl)-1-éthanone ; origine: Polak's Frutal Works, Hollande | |

L'addition de 100 parties en poids de (1-éthoxyéthoxy)cyclododécane à cette eau de toilette pour homme ajoute à la composition une note boisée-ambrée douce, tout en donnant du volume et une ténacité extraordinaire au parfum. En outre, l'addition du composé de l'invention a pour effet de faire ressortir de façon nettement plus intense la note masculine de la composition de base et la rend également plus riche que lorsqu'elle était dépourvue de (1-éthoxyéthoxy)cyclododécane.

### Exemple 5

### Préparation d'une composition parfumante pour une eau de toilette pour homme, à caractère aromatique

L'addition de 700 parties en poids de (1-éthoxyéthoxy)cyclododécane à la composition A telle que décrite dans l'exemple 3 a donné à cette composition de base une nouvelle dimension. En effet la fragrance de la nouvelle composition résultant de l'ajout dudit composé est devenue plus complexe, plus chaude, plus boisée et les notes aromatiques ont été nettement renforcées.

### Exemple 6

### Préparation d'une composition parfumante pour une base détergente à caractère fleuri et vert

On a préparé une composition parfumante de base pour un détergent à partir des ingrédients suivants :

| Ingrédients | Parties en poids |
|---|---|
| Acétate de cyclanol | 10 |
| Acétate de verdyle | 300 |
| Glycolate d'amyle allyle à 10% * | 60 |
| 4-Cyclohexyl-2-méthyl-2-butanol ¹⁾ | 100 |
| Dihydromyrcénol ²⁾ | 1600 |
| Polysantol ® ³⁾ | 20 |
| Galaxolide ® ⁴⁾ | 900 |
| Géranyle nitrile | 10 |
| Linalol | 150 |
| Méthylbutyrate d'isopropyle à 10% * | 150 |
| Myrcène à 10% * | 50 |
| 1-(5,5-diméthyl-1-cyclohexèn-1-yl)-4-penten-a-one ⁵⁾ à 1% * | 30 |
| α-Terpinéol | 40 |
| γ-Undécalactone | 10 |
| Verdox ® ⁶⁾ | 570 |
| Total | 4000 |

| | |
|---|---|
| * dans le dipropylène glycol 1) origine : Firmenich SA, Genève, Suisse 2) origine : International Flavors and Fragrances, USA 3) 3,3-diméthyl-5-(2,2,3-triméthyl-3-cyclopentèn-1-yl)-4-pentèn-2-ol ; origine : Firmenich SA, Genève, Suisse 4) 1,3,4,6,7,8-hexahydro-4,4,6,7,8,8-hexaméthyl-cyclopenta-γ-2-benzopyrane ; origine : International Flavors and Fragrances, USA 5) origine : Firmenich SA, Genève, Suisse 6) acétate de 2-tert-butyl-1-cyclohexyle ; origine: International Flavors and Fragrances, USA | |

L'addition de 900 parties en poids de (1-éthoxyéthoxy)cyclododécane à cette composition parfumante de base pour détergent a produit deux effets : d'une part, l'odeur de la composition après addition dudit composé est devenue plus riche, plus chaude, et a été appréciée pour sa très belle connotation boisée. En outre, après lavage, une évaluation effectuée par un panel de 22 parfumeurs a montré que la puissance et la douceur de la composition contenant le (1-éthoxyéthoxy)cyclododécane étaient bien supérieures à celles de la composition initiale. En effet sur les 22 parfumeurs, tous ont choisi la composition selon la présente invention, alors que 11 personnes du même panel ne sentaient pas le composé en tant que tel et qu'une personne le sentait très faiblement.

## Revendications

1. Utilisation du (1-éthoxyéthoxy)cyclododécane dans une composition parfumante, à titre d'agent fixateur et/ou amplificateur de parfum.

2. Utilisation selon la revendication 1, **caractérisée en ce que** le (1-éthoxyéthoxy)cyclododécane amplifie des notes odorantes de type aromatique et musqué.

3. Utilisation selon la revendication 1 ou 2, **caractérisée en ce que** la composition parfumante contient de 2 à 30% en poids de (1-éthoxyéthoxy)cyclododécane par rapport au poids total de la composition.

4. Composition parfumante ou article parfumé contenant le (1-éthoxyéthoxy)cyclododécane à titre d'agent fixateur et/ou amplificateur de parfum.

5. Article parfumé selon la revendication 4, choisi parmi un parfum ou une eau de toilette, une lotion après rasage, une préparation cosmétique, un savon, un shampooing ou après shampooing ou autre produit de soin capillaire, un gel de bain ou de douche, un déodorant corporel ou d'ambiance, un détergent, un adoucissant textile ou produit d'entretien.

## Claims

1. Use of (1-ethoxyethoxy)cyclododecane in a perfume composition as a perfume fixative and/or enhancer.

2. Use according to claim 1, **characterised in that** the (1-ethoxyethoxy)cyclododecane enhances aromatic and musky fragrance notes.

3. Use according to claim 1 or claim 2, **characterised in that** the perfume composition contains from 2 to 30% by weight of (1-ethoxyethoxy)cyclododecane relative to the total weight of the composition.

4. A perfume composition or perfumed article containing (1-ethoxyethoxy)cyclododecane as a perfume fixative and/or enhancer.

5. A perfumed article according to claim 4, selected from among a perfume or an eau de toilette, an aftershave lotion, a cosmetic preparation, a soap, a shampoo or hair conditioner or other hair care product, a bath or shower gel, a deodorant for personal use or air freshener, a detergent, a fabric softener or household product.

## Patentansprüche

1. Verwendung von (1-Ethoxyethoxy)cyclododecan in einer Parfümzusammensetzung als Fixiermittel und/oder Verstärker für Parfüm.

2. Verwendung nach Anspruch 1, **dadurch gekennzeichnet, dass** das (1-Ethoxyethoxy)cyclododecan die Geruchsnoten vom aromatischen und Moschustyp verstärkt.

3. Verwendung nach Anspruch 1 oder 2, **dadurch gekennzeichnet, dass** die Parfümzusammensetzung 2 bis 30 Gew.-% (1-Ethoxyethoxy)cyclododecan, auf das Gesamtgewicht der Zusammensetzung bezogen, enthält.

4. Parfümzusammensetzung oder parfümierter Gegenstand enthaltend das (1-Ethoxyethoxy)cyclododecan als Fixiermittel und/oder Verstärker für Parfüm.

5. Parfümierter Gegenstand nach Anspruch 4, ausgewählt unter einem Parfüm oder einem Eau de Toilette, einer After-Shave-Lotion, einer kosmetischen Zubereitung, einer Seife, einem Shampoo oder einer Spülung oder einem anderen Haarpflegeprodukt, einem Bade- oder Duschgel, einem Körper- oder Raumdeodorant, einem Detergens, einem Textilweichmacher oder Haushaltprodukt.
